(19) Europäisches Patentamt

European Patent Office

Office européen des brevets

(11) EP 1 020 182 A2

## (12) EUROPEAN PATENT APPLICATION

(43) Date of publication:
19.07.2000 Bulletin 2000/29

(51) Int. Cl.⁷: **A61K 9/20**, A61K 31/557

(21) Application number: 00300305.0

(22) Date of filing: 17.01.2000

(84) Designated Contracting States:
**AT BE CH CY DE DK ES FI FR GB GR IE IT LI LU MC NL PT SE**
Designated Extension States:
**AL LT LV MK RO SI**

(30) Priority: 18.01.1999 CA 2259727

(71) Applicant:
**SHERMAN, Bernard Charles
Willowdale, Ontario M2L 2K1 (CA)**

(72) Inventor:
**SHERMAN, Bernard Charles
Willowdale, Ontario M2L 2K1 (CA)**

(74) Representative:
**Votier, Sidney David
CARPMAELS & RANSFORD
43, Bloomsbury Square
London WC1A 2RA (GB)**

(54) **A two-layer pharmaceutical tablet comprising an nsaid and misoprostol**

(57)     A pharmaceutical tablet comprising misoprostol and an NSAID, said tablet comprising two layers wherein one of the misoprostol and NSAID is incorporated into only one layer and the other of the misoprostol and NSAID either is incorporated into the other layer or is incorporated between the two layers.

EP 1 020 182 A2

Printed by Xerox (UK) Business Services
2.16.7 (HRS)/3.6

**Description**

BACKGROUND OF THE INVENTION

The invention herein is directed to a pharmaceutical tablet which comprises both an NSAID and misoprostol.

Nonsteroidal anti-inflammatory drugs (NSAIDs) comprise a class of drugs which have long been recognized as having high therapeutic value especially for the treatment of inflammatory conditions such as exhibited in inflammatory diseases like osteoarthritis and rheumatoid arthritis. While the NSAIDs present a beneficial therapeutic value, they also exhibit undesirable side effects. An especially undesirable side effect of the administration of NSAIDs is the ulcerogenic effects generally associated with chronic use. NSAID induced ulcers in the stomach can be dangerous. Such ulcers generally exhibit few or no symptoms and may cause dangerous bleeding when undetected. In some instances, bleeding ulcers can prove fatal.

Certain prostaglandins have been shown to prevent NSAID induced ulcers. Misoprostol is a prostaglandin which has been accepted for use in the treatment of NSAID induced ulcers in many countries, including the United States.

It is desirable to provide a pharmaceutical composition which exhibits the beneficial properties of an NSAID and which also exhibits the beneficial properties of misoprostol for countering the ulcerogenic side effects attendent to NSAID administration.

This can be achieved by combining an NSAID and misoprostol in a single pharmaceutical tablet. However this is not easy to do, because misoprostol is highly unstable, and it is thus desirable not to have the misoprostol and NSAID mixed together, so as to prevent any deleterious effect of the NSAID on the stability of the misoprostol.

One solution to this problem, which is disclosed in US Patent 5601843, is to produce a tablet consisting of an inner core which comprises the NSAID and a mantle which surrounds the inner core and comprises the misoprostol. It is also disclosed that, in order to prevent contact between the misoprostol and the NSAID at the surface of the inner core, the inner core may be coated with an inert coating. Such coating may be an enteric coating, which also serves to reduce the likelihood of the NSAID dissolving in the stomach and thereby prevent exposing the stomach to the NSAID.

While the invention of US Patent 5601843 accomplishes its objective of separating the NSAID from the misoprostol, it has certain disadvantages. One disadvantage is the need to have a coating on the inner core in order to completely prevent contact between the NSAID in the inner core and the misoprostol in the mantle.

A second disadvantage is that the misoprostol is dispersed throughout the mantle, and is thus exposed to the environment at the surface of the tablet. This exposure increases the vulnerability of the misoprostol to degradation due to the effects of light or atmospheric oxygen and moisture.

The object of the present invention is to enable a pharmaceutical tablet that incorporates both an NSAID and misoprostol in a different manner from that disclosed in U.S. patent 5601843.

DESCRIPTION OF THE INVENTION

The present invention is a pharmaceutical composition in the form of a tablet comprising two layers. Either the misoprostol or the NSAID is incorporated within a first layer, and the other of the misoprostol and NSAID is either incorporated in a second layer or is incorporated between the first layer and second layer.

Pharmaceutical tablets are routinely made on a rotary tablet press. In the tabletting process, a mixture of materials in the form of a free flowing powder or granular mix is filled into dies as they pass under a feed frame. A punch protrudes into each die from beneath. After the die has passed under the feed frame, a second punch is then inserted into the die from above, and pressure is applied to the upper and lower punches to cause the powder or granular mix to be compressed into a tablet. The upper punch is then withdrawn, and the tablet is ejected from the die by raising the lower punch further into the die.

To make a two-layer tablet, a rotary tablet press with two feed frames is used. The material of which the first or lower layer of the tablet is to be comprised is filled into the die as it passes under the first feed frame, and the material of which the upper layer is to be comprised is added as the die passes under the second feed frame.

Compositions (i.e. tablets) of the present invention will be made on a rotary tablet press equipped as aforesaid with at least two feed frames so as to make tablets comprising at least two layers. The tablet press may optionally also be adapted with feed devices to feed pellets or smaller tablets into the dies so as to incorporate such pellets or smaller tablets into either or both layers of the tablet or to insert them between the two layers of the tablet.

Compositions of the present invention may be made in a number of variations, for example, as follows.

The misoprostol may be incorporated into the first or lower layer of the tablet in any of the following ways:

i) The misoprostol may be mixed with excipients (including a binder and a lubricant) and the resulting mixture fed into the die at the first feed frame so as to form the first or lower layer of the tablet. The misoprostol will then be distributed uniformly throughout the first layer.

ii) The misoprostol may be mixed with excipients and incorporated into small pellets in a separate operation done prior to the manufacture of the final two-layer tablet of the present invention. Those pellets may then be mixed with further excipients and the resulting mixture fed into the die at the first feed frame, so as to form the first or lower layer of the tablet. The misoprostol will then be located in pellets scattered in the first layer.

iii) The misoprostol may be mixed with excipients and incorporated into a small tablet (i.e. substantially smaller than the intended final two-layer tablet of the present invention) or into small pellets in a separate tabletting or pelletizing operation, done prior to the manufacture of the final two-layer tablet of the present invention. Either one such small tablet or a number of such small pellets comprising the misoprostol may then be fed into the die on the two-layer tablet press when the die is empty, before it passes under the first feed frame for addition of the powder or granular mix of which the first layer is to be comprised. As the die passes under the first feed frame, the powder or granular mix will cover and surround the small tablet or pellets comprising the misoprostol, so that upon subsequent compression to form the final two-layer tablet, the small tablet or pellets comprising the misoprostol will be incorporated into the first or lower layer.

Similarly, the NSAID may be incorporated into the second layer (or between the first and second layer) in any of the following ways:

i) The NSAID may be mixed with excipients (including a binder and a lubricant) and the resulting mixture fed into the die at the second feed frame so as to form the second or upper layer of the tablet. The NSAID will then be distributed uniformly throughout the second layer.
ii) The NSAID may be mixed with excipients and incorporated into small pellets in a separate operation done prior to the manufacture of the final two-layer tablet of the present invention. Those pellets may then be mixed with further excipients and the resulting mixture fed into the die at the second feed frame, so as to form the second or upper layer of the tablet. The NSAID will then be located in pellets scattered in the second layer.
iii) The NSAID may be mixed with excipients and incorporated into a small tablet (i.e. substantially smaller than the intended final two-layer tablet of the present invention) or into small pellets in a separate tabletting or pelletizing operation, done prior to the manufacture of the final two-layer tablet of the present invention. Either one such small tablet or a number of such small pellets comprising the NSAID may then be fed into the die on the two-layer tablet press after it passes under the first feed frame for addition of the powder or granular mix of which the first layer is to be comprised, but before it passes under the second feed frame. As the die passes under the second feed frame, the powder or granular mix will cover and surround the small tablet or pellets comprising the NSAID, so that upon subsequent compression to form the final two-layer tablet, the small tablet or pellets comprising the NSAID will be incorporated between the first and second layers.

It will be understood that the order of addition of the misoprostol and NSAID may be reversed, in which case the NSAID will be incorporated in the first or lower layer and the misoprostol will be incorporated either into the second layer or between the two layers.
The NSAID will preferably be piroxicam or diclofenac or a salt of diclofenac such as diclofenac sodium or diclofenac potassium. Most preferably, the NSAID will be diclofenac sodium.
Where diclofenac or a salt thereof is used, the amount per final tablet will preferably be from 25 to 75 mg. Preferably, the diclofenac or salt thereof will be incorporated into a small tablet or pellets as aforesaid and said small tablet or pellets will be coated with an enteric film coating to prevent the diclofenac or salt thereof from dissolving until after it has passed through the stomach and entered the small intestine. The enteric coating can be formulated with any suitable enteric coating polymer, many of which are known to those skilled in the art. Where piroxicam is used as the NSAID, the amount per tablet will preferably be 10 to 20 mg. Again the tablet containing piroxicam will also comprise usual tablet excipients.
The invention will be further understood from the following example, which is intended to be illustrative and not limiting of the scope of the invention.

EXAMPLE 1

Small tablets comprising misoprostol are made with a mixture of ingredients as follows:

|  | Amount per tablet |
|---|---|
| Misoprostol 1% dispersion in hydroxypropyl methylcellulose | 20.0 mg |
| Microcrystalline cellulose | 8.5 mg |
| Magnesium stearate | 0.5 mg |
| Croscarmellose sodium | 1.0 mg |
|  | 30.0 mg |

EXAMPLE 2

Small tablets comprising diclofenac sodium are made with a mixture of ingredients as follows:

|  | Amount per tablet |
|---|---|
| Diclofenac sodium | 50.0 mg |
| Microcrystalline cellulose | 24.0 mg |
| Magnesium stearate | 1.0 mg |
| Croscarmellose sodium | 5.0 mg |
|  | 80.0 mg |

These cores are then optionally enteric coated by spraying onto them a suspension or solution of an enteric coating polymer and a plasticizer.

EXAMPLE 3

A powder mixture for producing both the first and second layers of the final tablet is prepared as a mixture of 99.5% by weight microcrystalline cellulose and 0.5% magnesium stearate.

EXAMPLE 4

The final composition is then made on a two-layer tablet press.
The powder mixture from example 3 is loaded into the hoppers that supply both feed frames so that both layers of the final composition will be comprised of this same mixture. However, just before each die passes under the first feed frame, a tablet of example 1 is inserted into each die; and similarly just before each die passes under the second feed frame a tablet of example 2 is inserted into each die.
After the die passes out from under the second feed frame, an upper punch is inserted and pressure is applied between the upper and lower punches to compress the contents of the die into the final tablet of the invention. The upper punch is then withdrawn, and the lower punch is raised from below to eject the tablet.
It will be understood that the small tablet comprising misoprostol is thereby incorporated into the lower layer of the tablet and the small tablet comprising the diclofenac sodium is incorporated between the two layers of the tablet or at the bottom of the upper layer.

**Claims**

1.    A pharmaceutical composition in the form of a tablet comprising misoprostol and an NSAID and comprising two layers, wherein one of the misoprostol and the NSAID is incorporated into only one of the two layers and the other of the misoprostol and NSAID either is incorporated into the other of the two layers or is incorporated between the two layers.

2. A composition of claim 1 wherein the misoprostol is incorporated into only one of the two layers and the NSAID either is incorporated into the other of the two layers or is incorporated between the two layers.

3. A composition of claim 1 or 2 wherein the misoprostol is incorporated into a tablet that is substantially smaller than the composition and said tablet is incorporated into one of the two layers.

4. A composition of any of claims 1 to 3, wherein the NSAID is incorporated into a tablet that is substantially smaller than the composition and said tablet is incorporated into the one of the two layers or is incorporated between the two layers.

5. A composition of claim 4 wherein the NSAID is incorporated into a tablet that is substantially smaller then the composition and the said tablet is enteric coated.

6. A composition of any of claims 1 to 5 wherein the NSAID is diclofenac or a salt thereof.

7. A composition of claim 6 wherein the amount of diclofenac or salt thereof is from about 25 to about 75 mg.

8. A composition of claim 6 or 7 wherein the NSAID is diclofenac sodium.

9. A composition of any of claims 1 to 8 wherein the amount of misoprostol is about 200 micrograms.